**Europäisches Patentamt**

**European Patent Office**

**Office européen des brevets**

(19)

(11) Veröffentlichungsnummer: **0 001 806**
**B1**

(12) **EUROPÄISCHE PATENTSCHRIFT**

(45) Veröffentlichungstag der Patentschrift: **26.08.81**

(21) Anmeldenummer: **78101228.1**

(22) Anmeldetag: **25.10.78**

(51) Int. Cl.³: **A 61 N 1/08,** A 61 N 1/36,
G 05 F 1/58

(54) **Reizstromgerät.**

(30) Priorität: **07.11.77 DE 2749792**

(43) Veröffentlichungstag der Anmeldung:
**16.05.79 Patentblatt 79/10**

(45) Bekanntmachung des Hinweises auf die Patenterteilung:
**26.08.81 Patentblatt 81/34**

(84) Benannte Vertragsstaaten:
**BE DE FR GB NL SE**

(56) Entgegenhaltungen:
**DE - A - 1 589 021**
**DE - A - 1 589 520**
**FR - A - 1 603 649**
**FR - A - 2 332 034**
**FR - A - 2 339 906**
**US - A - 3 678 370**
**US - A - 3 713 020**
**US - A - 4 088 141**
**US - A - 4 102 347**

(73) Patentinhaber: **SIEMENS AKTIENGESELLSCHAFT**
**Berlin und München**
**Postfach 22 02 61**
**D-8000 München 22 (DE)**

(72) Erfinder: **Weigert, Kurt**
**Ernst-Heinkel-Weg 19**
**D-8500 Nürnberg (DE)**

Courier Press, Leamington Spa, England.

Reizstromgerät

Die Erfindung bezieht sich auf ein Reizstromgerät mit einem Stromformgenerator sowie einer Stromkonstantstufe für den Ausgangsstrom durch einen Belastungswiderstand, die mittels zugeordneter Gleichspannungsversorgung arbeitet, wobei Stromkonstantstufe und Gleichspannungsversorgung an einem Betriebsspannungsregler angeschltet sind, der in Abhängigkeit vom Belastungswiderstand bzw. von der Stromintensität durch den Belastungswiderstand die Betriebsspannung von einem Ausgangswert zu höheren Werten regelt.

Bekannte elektromedizinische Reizstromgeräte dieser Art haben eine stromkonstante Ausgangsstufe, die mit relativ hoher Betriebsspannung (bis zu einigen 100 V) arbeitet. Damit soll gewährleistet sein, daß auch bei extrem hohen Patientenwiderständen die zur Therapie bzw. Diagnostik erforderlichen Ströme mit dem entsprechend hohen Betriebsspannungsbedarf appliziert werden können.

Solche elektromedizinischen Reizstrom- geräte weisen daher regelmäßig Sicherheits- schaltungen auf, die verhindern sollen, daß unerwünschte Reizströme an den Patientenkörper gelangen. So wird beispielsweise mit der DE—A—15 89 520 eine Schaltung vorgeschlagen, die eine Reizstromapplikation dann unmöglich macht, wenn das Gerät zunächst mit bestimmter Stromintensität betrieben wurde, dan ausgeschaltet und anschließend wieder eingeschaltet wird. Es ist auch aus der DE—A—15 89 021 bekannt, den Reizstrom möglichst langsam und linear auf den gewünschten Intensitätswert hoch — bzw. von dort auf Nullwert herunter zu regeln. Das gleiche gilt aber sinngemäß auch für die aus der FR—A—16 03 649 sowie FR—A—23 32 034 bekannten Reizstromgeräte, die beide solche Fehlerschutzschaltungen für elektromedizinische Geräte beinhalten, welche bei Generierung von periodischen Reizstromimpulsen die Signal amplituden überwachen und bei überhöhter Amplitude das Gerät abschalten.

Bei üblicher Behandlung mit Reizstrom sind im allgemeinen die Patientenwiderstände relative niedrig (z. B. bei Anwendung großflächiger Elektroden); beim Applizieren wird in diesen Normalfällen also für die erforderlichen Ströme eine Betriebsspannung benötigt, die wesentlich niedriger liegt als die zur Verfügung stehende hohe Betriebsspannung. Diese Diskrepanz kann aber dann zu sehr unangenehmen Erscheinungen führen, wenn sich beispielsweise der Patientenwiderstand bei der Behandlung plötzlich ändert; dies gilt insbesondere für den Fall, daß sich die Stromzuführelektroden am Applikationsort gelöst haben und schließlich ganz abgefallen sind. Im Augenblick des Lösens verkleinert sich nämlich die Stromübergangsfläche rapide, so daß in Verbindung mit der Charakteristik einer Konstantstromquelle die Stromdichte auf der Haut am Applikationsort sehr hohe Werte annimmt. Dies führt zu einer sehr unangenehem schmerzhaften Reizung des behandelten Patienten. In vielen Fällen werden hierdurch auch direkte Verbrennungen der Haut verursacht. Sind die Zuführelektroden schließlich ganz abgefallen, so liegt zwischen den Elektroden, solange das Gerät noch in Betrieb ist und mit der vorher eingestellten Instensität weiter arbeitet, die volle gefährlich hohe Betriebsspannung.

Aufgabe der vorliegenden Erfindung ist es, bei einem Reizstromgerät der eingangs genannten Art dafür zu sorgen, daß unangenehme Strom- oder Spannungseffekte bei plötzlichen Abweichungen von den Normalbetriebsbedingungen mit Sicherheit vermieden werden. Insbesondere soll verhindert werden, daß es zu unangenehmen oder gar gefährlichen Rei- zungen kommt, wenn die Stromzuführ- elektroden sich lösen und abfallen. Ferner soll auch verhindert werden, daß die abgefallenen Elektroden die hohe Betriebsspannung führen.

Die Aufgabe wird erfindungsgemäß dadurch gelöst, daß der Spannungsregler die Betriebsspannung auf einen höheren Wert als den Aus- gangswert regelt, falls der Belastungswider- stand zunimmt und die Geschwindigkeit der Widerstandszunahme eine vorgebbare Schwelle nicht über schreitet, und daß der Spannungsregler falls die Geschwindigkeit der Widerstandszunahme diese Schwelle überschreitet, die Betriebsspannung auf einen Wert herunterregelt, der niedriger ist, als der bei Beginn dieser raschen Widerstandszunahme vorhandene Wert.

Beim Gerät nach der Erfindung liegt am Belastungswiderstand, d.h. beispielsweise am Patienten bei Reizstrombehandlung, im Normalfall immer relativ niedrige Betriebsspannung. Lediglich dann, wenn der Patientenwiderstand steigt oder beispielsweise auch die Stromintensität hochgeregelt wird, paßt sich die Betriebsspannung dem höheren Bedarfswert entsprechend automatisch an. Solange eine Änderung der genannten Größen relativ langsam erfolgt, folgt die Betriebsspannung diesen Änderungen in direkter Proportionalität. Erfolgt hingegen die Änderung sehr rasch, was z.B. beim Lösen und Abfallen von Elektroden oder sehr raschen Stromintensitätänderungen der Fall ist, so wird die Betriebsspannung nur sehr langsam hochgeregelt. Durch die dann erfolgende Umschaltung auf den niedrigeren Betriebsspannungswert werden unangenehm hohe Stromdichten verhindert und im Falle des Abfallens von Elektroden liegt an diesen Elektroden nur noch der niedrige Betriebsspannungswert.

Unangenehme Reizungen können, wie bereits angedeutet, auch durch zu rasches Hochregeln der Stromintensität an einem Intensitätseinstellglied verursacht werden. Obige Aufgabe wird erfindungsgemäß zusätzlich auch dadurch gelöst, daß der Spannungsregler die Betriebsspannung auf einen höher en Wert als den Ausgangswert regelt, falls die Stromintensität an einem Intensitätseinstellglied für die Stromkonstantstufe von einer Anfangsstromintensität zu höheren Werten geändert wird und die Geschwindigkeit der Stromintensitätsänderung eine vorgebbare Schwelle nicht überschreitet, und daß der Spannungsregler, falls die Geschwindigkeit der Stromintensitätsänderung diese Schwelle überschreitet, die Betriebsspannung auf einen Wert herunterregelt, der niedriger ist, als der bei Beginn dieser raschen stromintensitätsänderung vorhandene Wert. In diesem speziellen Falle tritt also genau das gleiche Ergebnis wie beispielsweise beim Lösen und anschließenden Abfallen von Elektroden ein, d.h. der Betriebsspannungswert wird ebenso wie im obigen Falle auf einen niedrigeren Wert geschaltet.

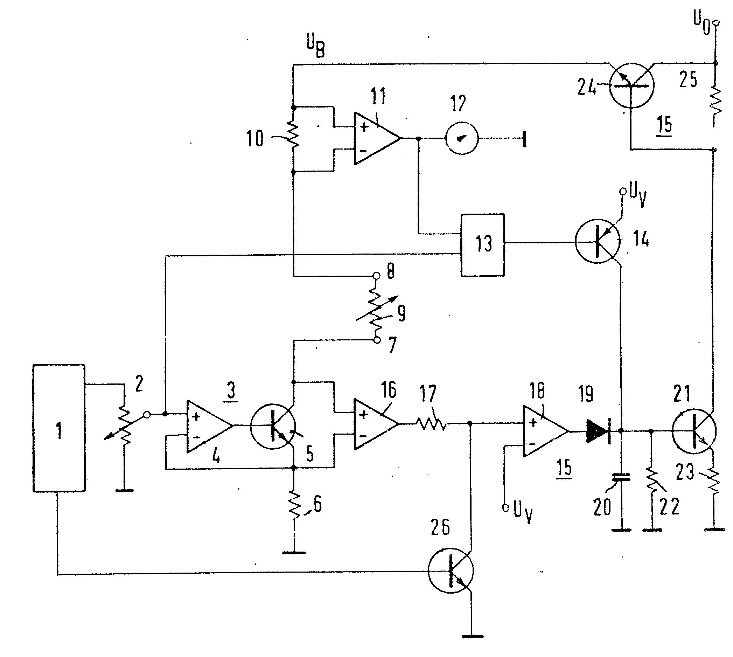

Weitere Vorteile und Einzelheiten der Erfindung ergeben sich aus der nachfolgenden Beschreibung eines Ausführungsbeispiels anhand der Zeichnung in Verbindung mit den Unteransprüchen.

Die Figur zeigt ein elektromedizinisches Reizstromgerät als Ausführungsbeispiel für die Erfindung im Prinzipschaltbild.

Im Prinzipschaltbild ist mit 1 ein Stromformgenerator für auszuwählende Reizströme bezeichnet. Der Stromformgenerator 1 steht über ein Stromintensitäteinstellglied 2 (Potentiometer) mit einer Stromkonstantstufe 3 in Verbindung, die in üblicher Weise aus Spannungsverstärker 4, Transistor 5 und Transistor-Emitter-Widerstand 6 besteht. Zwischen den Stromuführelektrodentroden 7 und 8 liegt gestrichelt angedeutet der variable Patientenwiderstand 9. Die Betriebsspannung für den Patientenstromkreis ist mit $U_B$ bezeichnet. Im Patientenstromkreis liegt ferner ein Meßwiderstand 10 dür den Istwert des Patientenstromes, der über eine Verstärkerstufe 11 einerseits an einem Anzeigegerät 12 für den Istwert des Patientenstromes und anderseits am einen Vergleichseingang eines Komparators 13 angeschaltet ist. Am anderen Vergleichseingang des Komparators 13 liegt der jeweilige Sollwert der Stromintensität, der durch die Ausgangsspannung des Intensitätseinstellgliedes 2 vorgegeben ist. Bei Abweichungen zwischen Ist- und Sollwert erzeugt der Komparator 13 ein Ausgangssignal, das den Transistor 14 in den leitenden Zustand schaltet, wodurch eine relativ niedrige Spannung $U_v$ ($U_v$ ungefähr 10 bis 20 V) in der dargestellten Weise in den Funktionskreis eines Betriebsspannungsreglers 15 eingeschaltet wird. Der Betriebsspannungsregler 15 beinhaltet einen Spannungsverstärker 16 für eine über der Kollektor-Emitter-Strecke des Transistors 5 in der Stromkonstantstufe 3 abgegriffene Spannung. Er umfaßt ferner den nachgeschalteten Widerstand 17 sowie ein Spannungseinstellglied 18 (Operationsverstärker), an dem eine Vergleichsspannung $U_v$ liegt (die im vorliegenden Ausführungsbeispiel der Spannung $U_v$ des Transistors 14 gleich ist). Auf den Spannungsverstärker 18 folgt über eine Diode 19 im Ankoppelpunkt des Transistors 14 eine Speicherkapazität 20 mit nachfolgendem ersten Regeltransistor 21, der mit Widerständen 22 und 23 beschaltet ist. Auf den Transistor 21 folgt ein zweiter Regeltransistor 24 mit dem Beschaltungswiderstand 25. Der Regeltransistor 24 liegt kollektorseitig an der Gleichspannungsversorgung $U_0$. Im Betriebsspannungsregler 15 ist darüber hinaus vor dem Spannungseinstellglied 18 auch noch ein Schalttransistor 26 angeordnet, der in Abhängigkeit von der Stromform des Stromgenerators 1 dem Spannungseinstellglied 18 nur dann Spannungssignale von der Stromkonstantstufe 3 zuschaltet, wenn Strommaxima im Belastungsstromkreis auftreten.

Die Funktionsweise der Prinzipschaltung ergibt sich wie folgt:

Bei der Intensität Null am Intensitätseinstellglied 2 erfolgt die Regelung der Betriebsspannung $U_B$ über den Betriebsspannungsregler 15 so, daß sich der Betriebsspannungswert $U_B$ auf den relativ niedrigen Betriebswert $U_v$ der Vergleichsspannung des Spannungseinstellgliedes 18 einregelt. Wird nun bei applizierten Elektroden 7 und 8 dem vorgebenen Patientenwiderstand 9 entsprechend die Intensität des Reizstromes im Patientenstromkreis langsam hochgeregelt, so steigt die Spannung über dem Patientenwiderstand, während der Spannungsabfall über dem Transistor 5 langsam abnimmt. Das langsame Abnehmen der Spannung über dem Transistor 5 bewirkt im Betriebsspannungsregler 15 eine langsame Regelung des Betriebsspannungswertes $U_B$ zu einem höheren Wert ($U_B > U_v$). Die Zeitkonstante der Betriebsspannungsregelung beträgt $\tau = C \cdot R \parallel R_{ET}$, wobei C die Kapazität des Kondensators 20 und $R \parallel R_{ET}$ der Widerstandswert der Parallelschaltung aus dem Widerstand 22 mit Widerstandswert R und dem Eingangswiderstand des Transistors 21 mit Widerstandswert $R_{ET}$ ist. Solange nun Änderungen der Intensität des Stromes im Patientenkreis sehr langsam erfolgen bzw. auch der Patientenwiderstand sich selbst nur sehr langsam ändert, erfolgt die Anpassung der Betriebsspannung $U_B$ mit etwa der Zeitkonstante dieser Änderung. Überschreitet jedoch die Änderung der Intensität oder auch die Änderung des Patientenwiderstandes eine vorgegebene Geschwindigkeitsschwelle — was immer dann gegeben ist, wenn sich die Elektroden 7 bzw. 8 am Patientenkörper lockern und abfallen oder wenn unbeabsichtigt die Stromintensität am Stromeinstellglied 2 zu rasch erhöht wird —, so wird durch den Betriebsspannungsregler 15 die Betriebsspannung $U_B$ weniger rasch zu höheren Werten geregelt als sich der beim Einstellen des Patientenstromes nötige Spannungsbedarf der Stromkonstantstufe vergrößert. Damit ergibt sich jedoch auch ein Mißverhältnis zwischen Ist-Wert des Stromes im Patientenstromkreis und dem mittels Intensitätsregler 2 eingestellten Soll-Wert. Der Ist-Wert sinkt, da aufgrund der sehr raschen Erhöhung des Patientenwiderstandes der Transistor 5 der Stromkonstantstufe in die Sättigung geht, wodurch die Spannung über dem Transistor 5 sehr rasch abfällt. Da das Absinken der Spannung über dem Transistor 5 mit sehr viel kleinerer Zeitkonstante als die Regelzeitkonstante des Regelkreises des Betriebsspannungsreglers 15 erfolgt, sinkt wegen der nur sehr verzögert nachfolgenden Betriebsspannung $U_B$ der Ist-Wert des Patientenstromes. Das Sinken des Ist-Werts, d.h. die Abweichung vom Soll-Wert, wird jedoch vom Komparator 13 erfaßt und mit der Abgabe eines Schaltsignales für den Transistor 14 beantwortet. Der Transistor 14 schaltet nun dem Kondensator 20 der Betriebsspannungsreglers 15 den Spannungswert $U_v$ auf, der dem Anfangsspannungswert bei der Intensitätseinstellung Null entspricht. Auf diese Weise stellt sich dann automatisch der Betriebsspannungswert $U_B = U_v$ an den Elektroden 7 und 8 ein. Mit der dargestellten Schaltung ist also erreicht, daß im Falle des sich Lösens und Abfallens der Elektroden 7 bzw. 8 vom Patientenkörper 9 wegen der sehr verzögerten Spannungsnachregelung der Ist-Wert des Reizstromes im Patientenstromkreis sehr rasch sinkt. Bei Nochkontakt der Elektroden 7 bzw. 8 mit dem Patientenkörper kurz vor dem Abfallen kommt es also mit Sicherheit auch nicht zu hohen Stromdichten beim Übergang auf die Haut; unangenehme oder schmerzhafte Reizung oder gar Verbrennungen der Haut werden somit vermieden. Sind die Elektroden schließlich abgefallen, so liegt an diesen Elektroden der sehr niedrige und damit auch ungefährliche Betriebsspannungswert $U_B = U_v$, der jenem bei der Intensitätseinstellung Null entspricht. Der Patient ist damit gegen unangenehme Auswirkungen bei Elektrodenlockerung oder Abfall gesichert. Dasselbe gilt auch für den Fall zu rascher Stromintensitätserhöhung. Wird nämlich das Einstellglied 2 unvorsichtigerweise zu rasch hochgeregelt, so ergibt sich der selbe Effekt wie bei zu rascher Widerstandsänderung. Der Transistor 5 wird rasch in die Sättigung getrieben, wodurch der Ist-Wert des Stromes im Patientenkreis rasch absinkt. Es tritt wiederum der Komparator 13 in Aktion, so daß über den Transistor 14 der Spannungswert $U_v$ in den Betriebsspannungsregelkreis 15 eingeschaltet wird. Auch bei zu schneller Stromerhöhung wird also der Betriebsspannungswert auf einen relativ niedrigen Wert geregelt. Auch von dieser Seite her kann der Patient also nicht mehr durch zu hohe stromdichten belästigt oder gar gefährdet werden.

**Patentansprüche**

1. Reizstromgerät mit einem Stromformgenerator (1) sowie einer Stromkonstantstufe (3) für den Ausgangsstrom durch einen Belastungswiderstand (9), die mittels zugeordneter Gleichspannungsversorgung ($U_o$) arbeitet, wobei Stromkonstantstufe (3) und Gleichspannungsversorgung ($U_o$) an einem Betriebsspannungsregler (15) angeschaltet sind, der in Abhängigkeit vom Belastungswiderstand (9) bzw. von der Stromintensität durch den Belastungswiderstand (9) bzw. von der Stromintensität durch den Belastungswiderstand (9) die Betriebsspannung ($U_B$) von einem Ausgangswert ($U_v$) zu höheren werten regelt, dadurch gekennzeichnet, daß der Spannungsregler (15) die Betriebsspannung ($U_B$) auf einen höheren Wert als den Ausgangswert regelt, falls der Belastungswiderstand (9) zunimmt und die Geschwindigkeit der Widerstandszunahme eine vorgebbare Schwelle nicht überschreitet, und daß der Spannungskregler (15), falls die Geschwindigkeit der Widerstandszunahme diese Schwelle überschreitet, die Betriebsspannung ($U_B$) auf einen Wert herunterregelt, der niedriger ist, als der bei Beginn dieser raschen Widerstandszunahme voerhandene Wert.

2. Reizstromgerät mit einem Stromformgenerator (1) sowie einer Stromkonstantstufe (3) für den Ausgangsstrom durch einen Belastungswiderstand (9), die mittels zugeordneter Gleichspannungsversorgung ($U_o$) arbeitet, wobei Stromkonstantstufe (3) und Gleichspannungsversorgung ($U_o$) an einem Betriebsspannungsregler (15) angeschaltet sind der in Abhängigkeit vom Belastungswiderstand (9) bzw. von der Stromintensität durch den Belastungswiderstand (9) die Betriebsspannung ($U_B$) voneinem Ausgangswert ($U_v$) zu höheren Werten regelt, dadurch gekennzeichnet, daß der Spannungsregler (15) die Betriebsspannung ($U_B$) auf einen höheren Wert als den Ausgangswert regelt, falls die Stromintensität an einem Intensitätseinstellglied (2) für die Stromkonstantstufe von einer Anfangsstromintensität zu höheren Werten geändert wird und die Geschwindigkeit der Stromintensitätsänderung eine vorgebbare Schwelle nicht überscheitet, und daß der Spannungsregler (15), falls die Geschwindigkeit der Stromintensitätsänderung diese Schwelle überschreitet, die Betriebsspannung ($U_B$) auf einen Wert herunterregelt, der niedriger ist, als der bei Beginn dieser raschen Stromintensitätsanderung vorhandene Wert.

3. Reizstromgerät nach Anspruch 1 oder 2, dadurch gekennzeichnet, daß die Anfangsstromintensität der Nullwert der Intensität ist.

4. Reizstromgerät nach Anspruch 1 oder 2, dadurch gekennzeichnet, daß der mittels Abwärtsregelung erreichte Wert der Betriebsspannung ($U_B$) der Ausgangswert ($U_v$) der

Betriebsspannung (U$_B$) ist.

5. Reizstromgerät nach Anspruch 1 oder 2, dadurch gekennzeichnet, daß der Spannungsregler (15) ein Spannungseinstellglied (18) umfaßt, das in Abhängigkeit von einer Vergleichsspannung (U$_V$) die Betriebsspannung auf die Vergleichsspannung regelt, wenn an einem Intensitätseinstellglied (2) für die Stromkonstantstufe (3) der Anfangsintensitätswert eingestellt ist, und daß dieser Vergleichsspannungswert (U$_V$) der Betriebsspannungsanfangswert ist.

6. Reizstromgerät nach Anspruch 5, dadurch gekennzeichnet, daß bei Spannungsabsenkung am Spannungseinstellglied (18) des Spannungsreglers (15), die entweder durch Stromintensitätsanstieg oder Anstieg des Belastungswiderstandes hervorgerufen wird, die Betriebsspannung vom Betriebsspannungsregler (15) zu höheren Werten geregelt wird.

7. Gerät nach Anspruch 5 oder 6, dadurch gekennzeichnet, daß die variable Eingangsspannung für das Spannungseinstellglied (18), die als Kriterium für Ämderungen des Belastungswiderstandes oder der Einstellung der Stromintensität dient, über ein Stromreglerglied (5), das Bestandteil der Stromkonstantstufe (3) ist, abgegriffen wird.

8. Gerät nach einem der Ansprüche 2 bis 7, dadurch gekennzeichnet, daß dem Stromreis für den Belastungswiderstand (9) sowie dem Stromintensitätseinstellglied (2) ein Komparator (13) zugeordnet ist, der den im Stromkreis für den Belastungswiderstand abgegriffenen Istwert des Stromes mit einem eingestellten Sollwert vergleicht und der dann, wenn der Istwert den Sollwert unterschreitet, den Spannungsregler (15) in dem Sinne einstellt, daß durch diesen der Betriebsspannungswert (U$_B$) auf den Ausgangswert (U$_V$) gestellt wird.

9. Gerät nach Anspruch 8, dadurch gekennzeichnet, daß der Istwert des Stromes durch den Belastungswiderstand (9) mittels Meßwiderstand (10) im Stromkreis für den Belastungswiderstand (9) abgegriffen wird.

10. Gerät nach Anspruch 8 oder 9, dadurch gekennzeichnet, daß an einem Ausgangskondensator (20) für das Spannungseinstellglied (18) im Spannungsregler (15) mittels eines Schaltglieds (14) eine Spannung (U$_V$) aufschaltbar ist dann, wenn am Komparator (13) der Istwert des Stromes im Belastungsstromkreis den Sollwert unterschreitet, wobei die zuzuschaltende Spannung mindestens dem Vergleichsspannungswert (U$_V$) des Spannungseinstellgliedes (18) entspricht oder größer als dieser ist.

11. Reizstromgerät nach Anspruch 10, dadurch gekennzeichnet, daß eine Diode (19) zur Entkopplung des Spannungseinstellgliedes (18) vom Ausgangskondensator (20) vorhanden ist, die bei Spannungen am Ausgangskondensator (20), die betragsmäßig größer sind als eine Ausgangsspannung am Spannungseinstellglied (18), gesperrt ist.

12. Reizstromgerät nach einem der Ansprüche 1 bis 11, dadurch gekennzeichnet, daß im Spannungsregler (15) vor dem Spannungseinstellglied (18) ein Schaltglied (26) angeordnet ist, das in Abhängigkeit von der Stromform des Stromgenerators (1) dem Spannungseinstellglied (18) nur dann Spannungssignale von der Stromkonstantstufe (3) zuschaltet, wenn Strommaxima im Belastungsstromkreis auftreten.

**Revendications**

1. Appareil à courant d'excitation, comportant un générateur de conformation du courant (1) ainsi qu'un étage à courant constant (3) pour le courant de sortie passant par une résistance de charge (9), et opérant à l'aide d'une alimentation en tension continue (U$_o$) associée, ledit étage à courant constant (3) et ladite alimentation en tension continue (U$_o$) étant reliés à un régulateur de la tension de service (15) qui, en fonction de la résistance de la charge (9) ou de l'intensité du courant passant par cette dernière, règle la tension de service (U$_B$) d'une valeur initiale (U$_V$) à des valeurs plus élevées, caractérisé par le fait que le régulateur de tension (15) règle la tension de service (U$_B$) à des valeurs qui sont supérieures à la valeur initiale dans le cas où la résistance d la charge (9) augmente et si la vitesse de l'accroissement de la résistance ne dépasse pas un seuil prédéterminé, et que, dans le cas où la vitesse de l'accroissement de la résistance dépasse ce seuil, le régulateur de tension (15) règle la tension de service (U$_B$) à une valeur qui est inférieure à la valeur qui était présente au début de cet accroissement rapide de la résistance.

2. Appareil à courant d'excitation, comportant un générateur de conformation du courant (1) ainsi qu'un étage à courant constant (3) pour le courant de sortie passant par une résistance de charge (9), et opérant à l'aide d'une alimentation en tension continue associée (U$_o$), ledit étage à courant constant (3) et ladite alimentation en tension continue (U$_o$) étant reliés à un régulateur de la tension de service (15) qui, en fonction de la résistance de la charge (9) ou de l'intensité du courant passant par la résistance de charge (9) règle la tension de service (U$_B$) d'une valeur initiale (U$_V$) à des valeurs plus élevées, caractérisé par le fait que le régulateur de tension (15) règle la tension de service (U$_B$) à des valeurs supérieures à la valeur de départ si l'intensité du courant est modifiée, au niveau d'un organe de réglage de l'intensité (2) pour l'étage à courant constant, d'une intensité initiale vers des valeurs plus élevées et si la vitesse de la modification de l'intensité ne dépasse pas une valeur prédéterminée, et que, dans le cas où la vitesse de la modification de l'intensité du courant dépasse ce seuil, le régulateur de tension (15) règle la tension de service (U$_B$) à une valeur qui est inférieure à la valeur qui était présente au début de cet accroissement très rapide de la modification de l'intensité du courant.

3. Appareil à courant d'excitation selon la revendication 1 ou 2, caractérisé par le fait que l'intensité initiale du courant correspond à la valeur nulle de l'intensité.

4. Appareil à courant d'excitation selon la revendication 1 ou 2, caractérisé par le fait que la valeur de la tension de service $(U_B)$ qui est atteinte à l'aide du réglage vers les valeurs inférieures, est égale à la valeur initiale $(U_V)$ de la tension de service $(U_B)$.

5. Appareil à courant d'excitation selon la revendication 1 ou 2, caractérisé par le fait que le régulateur de tension comprend un organe de réglage de la tension (18) qui régle, en fonction d'une tension de référence $(U_V)$, la tension de service au niveau de la tension de référence si, au niveau d'un organe de réglage de l'intensité (2) pour l'étage à courant constant (3), la valeur de l'intensité initiale est réglée, et que cette valeur de la tension de référence $(U_V)$ est égale à la valeur initiale de la tension de service.

6. Appareil à courant d'excitation selon la revendication 5, caractérisé par le fait que lors de l'abaissement de la tension au niveau de l'organe de réglage de la tension (18) du régulateur de tension (15), provoqué soit par un accroissement de l'intensité du courant, soit par un accroissement de la résistance de la charge, la tension de service est réglée à des valeurs plus élevées à l'aide du régulateur de tension de service (15).

7. Appareil à courant d'excitation selon la revendication 5 ou 6, caractérisé par le fait que la tension d'entrée variable pour l'organe de réglage de la tension (18), qui sert de critère pour des variations de la résistance de charge ou qui sert au réglage de l'intensité du courant, est prélevée par l'intermédiaire d'un organe de réglage de courant (5) formant un elément constitutif de l'étage à courant constant (3).

8. Appareil à courant d'excitation selon l'une des revendications 2 à 7, caractérisé par le fait qu'au circuit du courant pour la résistance de charge (9) ainsi qu'à l'organe de réglage de l'intensité du courant (2), est associé un comparateur (13) qui compare la valeur instantanée prélevée dans le circuit de courant pour la résistance de charge avec la valeur de référence réglée, ledit comparateur réglant, lorsque la valeur instantanée dépasse la valeur de référence, le régulateur de tension (15) dans un sens tel que, par ledit régulateur de tension, la valeur de la tension de service $(U_B)$ est ajustée à la valeur de départ $(U_V)$.

9. Appareil à courant d'excitation selon la revendication 8, caractérisé par le fait que la valeur instantanée du courant passant par la résistance de change (9) est prélevée à l'aide d'une résistance de mesure (10) dans le circuit du courant pour la résistance de charge (9).

10. Appareil à courant d'excitation selon la revendication 8 ou 9, caractérisé par le fait qu'au niveau d'un condensateur de sortie (20) pour l'organe de réglage de la tension (18), situé dans le régulateur de tension (19), et au moyen d'un élément de commutation (14), une tension $(U_V)$ peut être appliquée lorsque, au niveau du comparateur (13), la valeur instantanée du courant dans le circuit de charge se situe en-dessous de la valeur de référence, ladite tension à appliquer correspondant au moins à la valeur de la tension de référence $(U_V)$ de l'organe de réglage de la tension (18) ou lorsqu'elle lui est supérieure.

11. Appareil à courant d'excitation selon la revendication 10, caractérisé par le fait qu'il est prévu une diode (19) pour découpler l'organe de réglage de la tension (18) du condensateur de sortie (20), ladite diode étant bloquée pour des tensions aux bornes du condensateur (20) qui ont des valeurs supérieures à une tension de sortie au niveau de l'organe de réglage de la tension (18).

12. Appareil à courant d'excitation selon l'une des revendications 1 à 11, caractérisé par le fait que l'on prévoit dans le régulateur de tension (15), en amont de l'organe de réglage de la tension (18), un organe de commutation qui, en fonction de la forme du courant du générateur de courant (1) n'applique, à l'organe de réglage de la tension (18), des signaux de tension provenant de l'étage à courant constant (3) que lorsque des maxima de courant apparaissent dans le circuit de la charge.

**Claims**

1. A stimulation current device comprising a current waveform generator (1) and a constant current stage (3) supplying output current to a load impedance (9), which constant current stage operates by means of an assigned d.c. voltage supply $(U_o)$, wherein the constant current stage (3) and the d.c. voltage supply $(U_o)$ are connected to an operating voltage regulator (15) which adjusts the operating voltage $(U_B)$ from an initial level $(U_V)$ to higher levels in dependence upon the load impedance value (9) or upon the intensity of the current passing through the load impedance (9), characterised in that the voltage regulator (15) adjusts the operating voltage $(U_B)$ to a higher level than the initial level if the load impedance (9) increases in value and the rate of increase in the impedance value does not exceed a predeterminable threshold, and that if the rate of increase in the impedance value exceeds this threshold, the voltage regulator (15) regulates the operating voltage $(U_B)$ down to a level which is lower than the level present at the start of this rapid increase in impedance value.

2. A stimulation current device which comprises both a current waveform generator (1) and a constant current stage (3) supplying output current to a load impedance (9), which constant current stage operates by means of assigned direct voltage supply $(U_o)$, wherein the

constant current stage (3) and d.c. voltage supply ($U_0$) are connected to an operating voltage regulator (15) which adjusts the operating voltage ($U_B$) from an initial level ($U_V$) to higher levels in dependence upon the value of the load impedance (9) or the intensity of the current flowing through the load impedance (9), characterised in that the voltage regulator (15) adjusts the operating voltage ($U_B$) to a higher level than the initial level if the current intensity at an intensity adjusting element (2) is changed from an initial current intensity to higher values for the constant current stage and the rate of change in current intensity does not exceed a predeterminable threshold, and that if the rate of change in current intensity exceeds this threshold, the voltage regulator (15) regulates the operating ($U_B$) down to a level which is lower than the level present at the start of this rapid change in current intensity.

3. A stimulation current device as claimed in Claim 1 or Claim 2, characterised in that the initial current intensity is zero.

4. A stimulation current device as claimed in Claim 1 or Claim 2, characterised in that the level of the operating voltage ($U_B$) reached following downward regulation is the initial level ($U_V$) of the operating voltage ($U_B$).

5. A stimulation current device as claimed in Claim 1 or Claim 2, characterised in that the voltage regulator (15) comprises a voltage adjusting element (18) which, in dependence upon a comparison voltage ($U_V$), adjusts the operating voltage to the comparison voltage when the initial intensity value adjusted by an intensity adjusting element (2) for the constant current stage (3), and that this comparison voltage level ($U_V$) is the initial level of the operating voltage.

6. A stimulation current device as claimed in Claim 5, characterised in that if there is a voltage drop at the voltage adjusting element (18) of the voltage regulator (15), caused either by an increase in the current intensity or an increase in the load impedance value, the operating voltage level is adjusted to higher levels by the operating voltage regulator (15).

7. A device as claimed in Claim 5 or Claim 6, characterised in that the variable input voltage for the voltage adjusting element (18), which serves as a criterion for the changes in the value of the load impedance or in the adjustment of the current intensity, is tapped by means of a current regulator element (5) which is a component part of the constant current stage (3).

8. A device as claimed in one of Claims 2 to 7, characterised in that both the electric circuit for the load impedance (9) and the current adjusting element (2) are allocated a comparator (13) which compares the actual value of the current tapped in the electric circuit for the load impedance with a set theoretical value and which then adjusts the voltage regulator (15) if the actual value undershoots the theoretical value to the effect that by means of the latter, the operating voltage ($U_B$) is adjusted to the initial level ($U_V$).

9. A device as claimed in Claim 8, characterised in that actual value of the current through the load impedance (9) is tapped by means of a measuring impedance (10) in the electric circuit for the load impedance (9).

10. A device as claimed in Claim 8 or Claim 9, characterised in that at an output capacitor (20) for the voltage adjusting element (18) in the voltage regulator (15) there can be applied a voltage ($U_V$) by means of a switching element (14) when the actual value of the current in the load circuit undershoots the theoretical value at the comparator (13), the voltage to be applied being identical to at least the comparison voltage level ($U_V$) of the voltage adjusting element (18) or being larger than the comparison voltage level ($U_V$).

11. A stimulation current device as claimed in Claim 10, characterised in that a diode (19) is provided for decoupling the voltage adjusting element (18) from the output capacitor (20), which diode is blocked in the case of voltage levels at the output capacitor (20) being greater than an output voltage level at the voltage adjusting element (18).

12. A stimulation current device as claimed in one of Claims 1 to 11, characterised in that in the voltage regulator (15) before the voltage adjusting element (18) there is arranged a switching element (26) which in dependence upon the current form of the current generator (1) connects voltage signals from the constant current stage (3) to the voltage adjusting element (18) only if current maxima occur in the load circuit.